# EUROPÄISCHE PATENTANMELDUNG

(11) **EP 0 625 498 A1**
(43) Veröffentlichungstag der Anmeldung: **23.11.1994**
(21) Anmeldenummer: 94107476.7
(22) Anmeldetag: 13.05.1994
(51) Int. Cl.: C07C 51/10, C07C 65/11

(54) **Verfahren zur Herstellung von aromatischen Hydroxycarbonsäuren**

(30) Priorität: 21.05.1993 DE 4316937
(71) Anmelder: HOECHST AKTIENGESELLSCHAFT, D-65929 Frankfurt am Main (DE)
(72) Erfinder: Rittner, Siegbert, Dr., D-64546 Mörfelden (DE); Rüffer, Hans-Martin, Dr., D-65719 Hofheim (DE); Schmid, Jörg, Dr., D-65817 Eppstein (DE); Wisser, Thomas, Dr., D-65552 Limburg (DE)

(57) **Zusammenfassung**

Ein Verfahren zur Herstellung von aromatischen Hydroxycarbonsäuren durch Umsetzung von Alkaliphenolaten oder Alkalinaphtholaten mit Alkalicarbonaten und Kohlenmonoxid und anschließendem Ansäuern, dadurch gekennzeichnet, daß die festen Edukte Alkalicarbonat und/oder Alkali-phenolat oder -naphtholat in Form einer oder mehrerer Dispersionen in einer inerten organischen Flüssigkeit in die Reaktionsmischung zudosiert werden.

## Beschreibung

Die Erfindung betrifft ein Verfahren zur Herstellung von aromatischen Hydroxycarbonsäuren oder deren Salzen durch Umsetzung eines entsprechenden Alkaliphenolats oder Naphtholats mit Kohlenmonoxid und einem Alkalicarbonat.

Aromatische Hydroxycarbonsäuren, wie 2-Hydroxy-naphthalin-6-carbonsäure, sind wichtige Zwischenprodukte z. B. bei der Herstellung von Farbstoffen, Polyestern, Textilhilfsmitteln und Arzneimitteln (siehe beispielsweise EP-A 0 292 955 und US 4,393,191).

Technisch erfolgt die Herstellung solcher Verbindungen vielfach nach der Kolbe-Schmitt-Reaktion, d. h. durch Umsetzung eines entsprechenden Alkaliphenolats oder Naphtholats mit Kohlendioxid (siehe z. B. EP-A 0 327 221 bzw. US 4,966,992). Die Ausbeuten dieser Verfahren sind jedoch noch verbesserungsfähig.

Ein alternatives Herstellungsverfahren für aromatische Hydroxycarbonsäuren ist in GB 1 155 776 (≙ US 3,655 744) beschrieben. Dabei werden bestimmte aromatische Alkali- oder Erdalkaliphenolate bzw. Naphtholate mit Alkali-oder Erdalkalicarbonaten, -carboxylaten oder -dicarboxylaten in Gegenwart von Kohlenmonoxid umgesetzt. Die Ausbeuten und insbesondere die Selektivitäten des Verfahrens sind ebenfalls nicht für alle Bereiche ausreichend.

Wünschenswert war es also, das in der britischen Patentanmeldung beschriebene Verfahren in Bezug auf Ausbeuten und Selektivität der Reaktion zu verbessern. Weiterhin war es wünschenswert, das Verfahren so zu gestalten, daß es in einfacher Weise kontinuierlich durchgeführt werden kann.

So werden in GB 1 155 776 zwar die Möglichkeiten der kontinuierlichen Reaktionsführung, der Zugabe von Lösemitteln und der Zu- bzw. Abführung von festen Stoffen als Aufschlämmung erwähnt, konkrete Beispiele werden aber nicht gegeben.

Es wurde nun überraschend gefunden, daß Alkaliphenolate und Naphtholate und/oder Alkalicarbonate mit inerten organischen Flüssigkeiten bei Raumtemperatur stabile fließ- und pumpfähige Dispersionen bilden. Es zeigte sich, daß durch Umsetzung der festen Edukte in Form solcher Dispersionen, die Selektivität der Reaktion deutlich erhöht werden kann, insbesondere wenn die Zugabe zu der Reaktionsmischung über den gesamten Reaktionsverlauf ausgedehnt wird.

Gegenstand der Erfindung ist daher ein Verfahren zur Herstellung von aromatischen Hydroxycarbonsäuren durch Umsetzung von Alkaliphenolaten oder Naphtholaten mit Alkalicarbonaten und Kohlenmonoxid und anschließendes Ansäuern, dadurch gekennzeichnet, daß die festen Edukte Alkalicarbonat und Phenolate oder Naphtholate in Form einer Dispersion mit einer inerten organischen Flüssigkeit in die Reaktionsmischung absatzweise oder kontinuierlich eingebracht werden.

Das erfindungsgemäße Verfahren ermöglicht die Herstellung aromatischer Hydroxycarbonsäuren in guten Ausbeuten und hoher chemischer Selektivität. Auf zusätzliche Maßnahmen zur Steigerung der Selektivität, wie sie für die Kolbe-Schmitt-Reaktion beispielsweise in EP-A 0 053 824, EP-A 0 081 753 und EP-A 0 254 596 und für die Reaktion mit CO und Carbonat beispielsweise in WO-91/11422 beschrieben sind, kann verzichtet werden.

Wärmelabile und oxidationsempfindliche Stoffe, wie Phenolate und Naphtholate, können bei Raumtemperatur absatzweise oder kontinuierlich zudosiert werden und müssen nicht wie bisher bei hohen Temperaturen, unter Inkaufnahme von Zersetzungsprodukten, als Schmelze zudosiert werden.

Auch hochschmelzende Feststoffe, wie Kaliumcarbonat, können kontinuierlich zudosiert werden.

Ebenfalls Gegenstand der Erfindung sind daher Dispersionen aus einem Alkalicarbonat und/oder einem Phenolat oder Naphtholat und einer inerten organischen Flüssigkeit.

Als Phenolate oder Naphtholate werden bevorzugt Verbindungen der Formel (I) eingesetzt 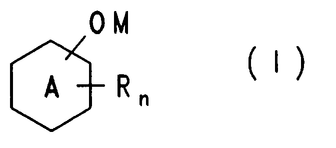
worin die Symbole und Indizes folgende Bedeutungen haben: 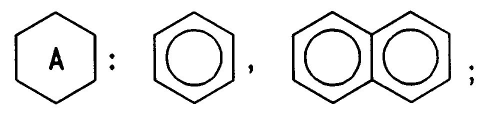
- M :: Li, Na, K;
- R :: unabhängig voneinander, OM, COOM, F, Cl, Br, I, NH₂, CF₃, eine Alkylgruppe mit 1 bis 6 C-Atomen, eine Alkoxygruppe mit 1 bis 6 C-Atomen;
- n :: 0, 1, 2, 3, 4.

Vorzugsweise sind:
- M :: Na, K;
- R :: -OM, -COOM, eine Alkylgruppe mit 1 bis 6 C-Atomen;
- n :: 0, 1, 2.

Besonders bevorzugt sind:
- M :: K;
- R :: COOM, -CH₃;
- n :: 0,1.

Insbesondere bevorzugt als Verbindung der Formel (I) sind Natrium-β-naphtholat und Kalium-β-naphtholat.

Die erfindungsgemäß eingesetzten Alkaliphenolate und Naphtholate können durch Umsetzung der entsprechenden Phenole oder Naphthole mit basischen Alkaliverbindungen, wie Natriumhydroxid und Kaliumhydroxid, hergestellt werden.

Als Carbonat können in den erfindungsgemäßen Verfahren alle Alkalicarbonate und Hydrogencarbonate, d. h. Li₂CO₃, LiHCO₃, Na₂CO₃, NaHCO₃, K₂CO₃, KHCO₃, Rb₂CO₃, RbHCO₃, Cs₂CO₃ und CsHCO₃, eingesetzt werden. Bevorzugt ist der Einsatz von Na₂CO₃ und K₂CO₃, insbesondere von K₂CO₃.

Die erfindungsgemäß verwendeten Carbonate sollten vorzugsweise wenig Feuchtigkeit enthalten, insbesondere weniger als 0,5 Gew.-% Wasser.

Bezogen auf das eingesetzte Phenolat oder Naphtholat sollten mindestens äquivalente Mengen von Carbonat eingesetzt werden. Im allgemeinen beträgt das stöchiometrische Verhältnis von Phenolat oder Naphtholat zu Carbonat 1:1 bis 1:4, bevorzugt 1:1 bis 1:3, besonders bevorzugt 1:1 bis 1:1,5.

Die Korngröße der eingesetzten festen Edukte (Phenolat, Naphtholat und Carbonat) liegt vorzugsweise unter 50 µm, besonders bevorzugt unter 10 µm, insbesondere zwischen 0,1 und 10 µm.

Als Mahlaggregate für die festen Edukte eignen sich alle Zerkleinerungsmaschinen z. B. Mühlen, die spröde Feststoffe zu derartigen Korngrößen zerkleinern können. Besonders gute Ergebnisse werden erzielt, wenn die Feststoffe direkt mit dem Dispergiermittel naß, z. B. in Kugelmühlen, vermahlen werden.

Als Dispergiermittel zur Herstellung der erfindungsgemäßen Dispersion werden unter den Reaktionsbedingungen inerte, temperaturstabile, flüssige Stoffe oder Stoffgemische eingesetzt, wie aliphatische, alicyclische oder aromatische Kohlenwasserstoffe aus der Erdöldestillation. Insbesondere geeignet sind Leichtöl, Schweröl, vorzugsweise Kerosin, Aromaten und deren Alkylderivate, wie Toluol, Xylol, Isopropyl- oder Diisopropylnaphthalin, Diphenyl, Alkyldiphenyle, Triphenyl und Alkyltriphenyle, sowie aliphatische und aromatische Etherverbindungen und deren Alkylderivate, wie Diphenylether, Anisol, Dicyclohexylether, und Mischungen aus denselben. Die Dispergiermittel stören die Reaktion nicht und können aus dem Reaktor abdestilliert werden oder im Anschluß an die Reaktion durch Dekantieren oder Destillieren von der Reaktionsmasse abgetrennt werden. Sie haben den Vorteil, daß sie nach Entfernung von möglicherweise gelösten oder mitgeschleppten Bestandteilen wie β-Naphthol oder Wasser, wieder zur Erzeugung frischer Dispersionen eingesetzt werden können.

Die Eindosierung der Phenolat bzw. Naphtholate sowie des Carbonats kann in einer stofflich gemeinsamen Dispersion oder in stofflichen Einzeldispersionen erfolgen, wobei letztere Möglichkeit eine größere Variationsbreite für die Reaktionsführung eröffnet.
Zum Einbringen in die Reaktionsmischung sind alle Dosiereinrichtungen geeignet, die Suspensionen oder Pasten gegen den Druck im Reaktor fördern können, wie Kolbenpumpen, Membranpumpen, Extruder und Exzenterschneckenpumpen.

Die Zusammensetzung der Dispersion kann gewichtsmäßig in weiten Grenzen liegen und richtet sich danach, ob sie als leichtfluides Medium oder in pastöser Form gefördert werden soll. In Bezug auf Alkaliphenolat oder Naphtholat eignet sich ein Gewichtsanteil von 5 bis 70 Gew.-%, vorzugsweise 25 bis 60 Gew.-%, insbesondere auch zwischen 40 bis 55 Gew.-%, in Bezug auf die Masse der Dispersion. Kaliumcarbonat kann in den Grenzen von 5 bis 65 Gew.-%, vorzugsweise 20 bis 55 Gew.-%, insbesondere im Bereich von 25 bis 40 Gew.-%, bezogen auf das Gesamtgewicht der Dispersion, eingesetzt werden. Bei Feststoffgehalten unter 25 Gew.-% ist in manchen Fällen ein Absetzverhalten zu beobachten, das bereits durch moderates Rühren oder Pumpen verhindert werden kann.

Die als Dispergiermittel beschriebenen organischen Flüssigkeiten können der Reaktion auch als zusätzliche Löse- und Verdünnungsmittel zugesetzt werden, vorzugsweise in Mengen von 10 bis 300 Gew.-%, besonders bevorzugt 50 bis 150 Gew.-%, bezogen auf die festen Edukte. Analog dem in der EP-A 0 081 753 beschriebenen Verfahren zur Abtrennung von Nebenprodukten bei der Synthese von 2-Hydroxy-naphthalin-6-carbonsäure nach Kolbe-Schmitt kann das Dispergiermittel und/oder ein zusätzliches Verdünnungsmittel auch zur Abtrennung von Nebenprodukten verwendet werden.

Für manche Reaktionen ist es vorteilhaft, wie in der WO 91/11422 beschrieben, dem Reaktionsansatz Kaliumformiat zuzusetzen, das unter den üblichen Reaktionsbedingungen als dünnflüssige, klare Schmelze vorliegt. Es dient als Löse- und Verdünnungsmittel und wird vorzugsweise in Mengen von 50 bis 1000 Gew.-%, besonders bevorzugt 100 bis 300 Gew.-%, bezogen auf die festen Edukte, eingesetzt.

Das erfindungsgemäße Verfahren wird in Gegenwart von CO durchgeführt. Das CO kann als Gasatmosphäre über der Reaktionsmischung vorliegen oder auf bzw. auch direkt in die Mischung gedrückt werden. Um die Reaktion vollständig ablaufen zu lassen, werden mindestens stöchiometrische Mengen, bezogen auf das Phenolat oder Naphtholat, an CO benötigt.

Das erfindungsgemäße Verfahren wird bei einem Druck von 1 bis 150 bar, vorzugsweise 5 bis 100 bar und besonders bevorzugt von 10 bis 30 bar, betrieben, wobei der Druck bei der Reaktionstemperatur gemeint ist.

Es kann technisches Kohlenmonoxid verwendet werden, d. h., geringe Mengen an Fremdgasen, wie N₂, CH₄, CO₂, H₂, sind unkritisch.

Die Reaktionstemperatur kann je nach Eigenschaften der Edukte, Produkte und Löse- oder Dispergiermittel in weiten Grenzen variiert werden. Im allgemeinen wird bei Temperaturen von 150 bis 400°C, bevorzugt von 150 bis 350°C, ganz besonders bevorzugt von 200 bis 350°C, gearbeitet.
Die Reaktionsdauer beträgt vorzugsweise zwischen 1 und 40 Stunden.

Zur Durchführung des erfindungsgemäßen Verfahrens können unterschiedliche apparative Ausführungsformen zum Einsatz gelangen, so kann beispielsweise als Reaktor ein Druckgefäß oder Kneter verwendet werden, der mit einem zur Durchmischung und CO-Begasung effizienten Rührorgan ausgerüstet und mit einer Dispersionseinspeisung verbunden ist. Dabei kann die Dispersion entweder getaktet, zugepumpt oder zugedrückt bzw. kontinuierlich eindosiert werden.

In einer bevorzugten Variante des erfindungsgemäßen Verfahrens werden Phenolat oder Naphtholat, Carbonat und Dispergiermittel vermahlen, und die so erhaltene Dispersion wird kontinuierlich oder getaktet in einen vorgeheizten, mit CO und gegebenenfalls mit Lösungsmittel und Carbonat beschickten Reaktor eingebracht. Nach Beendigung der Reaktion wird das entstandene Alkalisalz in üblicher Weise in die Säure überführt und gegebenenfalls nach bekannten Verfahren gereinigt.

Das erfindungsgemäße Verfahren kann diskontinuierlich, semikontinuierlich oder kontinuierlich betrieben werden.

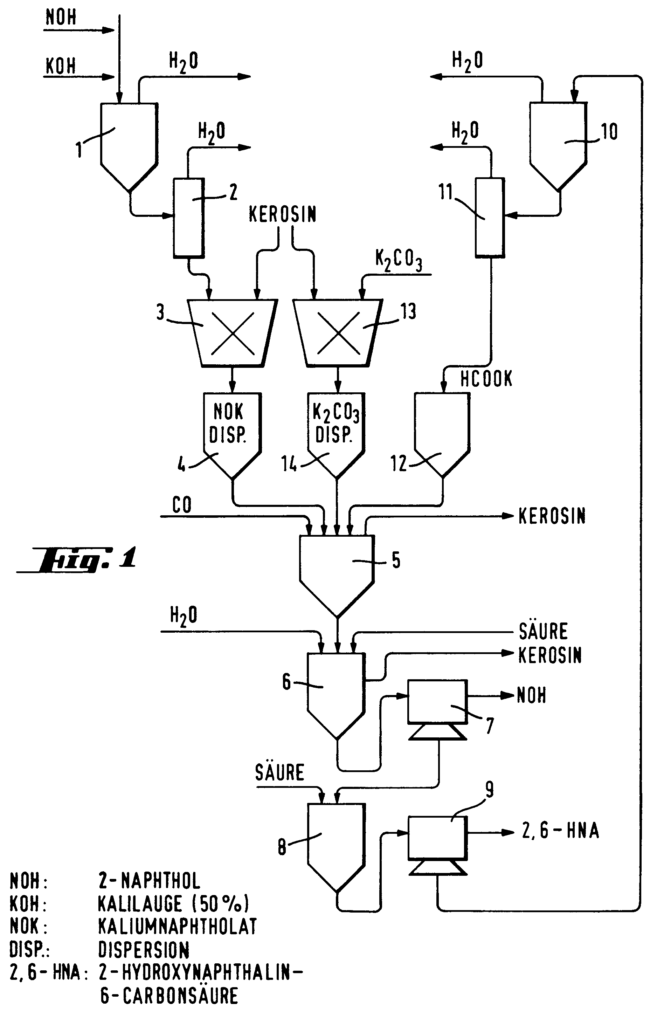

In Figur 1 ist eine mögliche Variante zur apparativen Durchführung des erfindungsgemäßen Verfahrens dargestellt.

2-Naphthol wird zusammen mit Kalilauge im Eindampfer (1) entwässert und im Trockner (2) von Restfeuchte befreit. Die gebildete Kaliumnaphtholatschmelze wird anschließend unter Kühlung in der Mühle (3) mit Kerosin zur Dispersion vermahlen und im Tank (4) bevorratet. Die gegebenenfalls zurückgeführte und im Eindampfer (10) und Trockner (11) entwässerte Kaliumformiatschmelze wird über das Dosiergefäß (12) im Reaktor (5) vorgelegt. Die Suspensionen von Kaliumnaphtholat und Kaliumcarbonat werden unter CO-Druck kontinuierlich zugeführt (hergestellt durch Vermahlen mit Kerosin in der Mühle (13) und bevorratet im Tank (14)). Nach erfolgter Reaktion wird die Reaktionsschmelze im Rührbehälter (6) in Wasser aufgenommen, Kerosin als Oberphase abdekantiert und mit Schwefelsäure neutral gestellt. Das gefällte 2-Naphthol wird in der Zentrifuge (7) abgetrennt. Die Ansäuerung und Fällung der 2-Hydroxy-naphthalin-6-carbonsäure geschieht analog in (8) und (9).

Die nach dem erfindungsgemäßen Verfahren hergestellten aromatischen Hydroxycarbonsäuren sind wichtige Zwischenprodukte zur Herstellung von Polyestern, Azofarbstoffen und Arzneimitteln.

Insbesondere stellt 2-Hydroxy-naphthalin-6-carbonsäure nicht nur einen wertvollen Synthesebaustein für Farbstoffe, Textilhilfsmittel und Arzneimittel dar (siehe z. B. EP-A 0 292 955 A), sondern insbesondere auch ein wichtiges Monomeres zur Herstellung von flüssigkristallinen Polymeren mit überragenden Eigenschaften (siehe z. B. US-PS 4 393 191).

Die nachfolgenden Beispiele dienen zur Erläuterung der vorstehend beschriebenen Erfindung. Die Teile sind Gewichtsteile, die Prozentangaben stellen Gewichtsprozente dar, sofern nicht anders vermerkt. Die Gewichtsteile verhalten sich zu Volumenteilen wie Kilogramm zu Liter.

### Beispiele

### Beispiel 1

Ein Edelstahl-Druckautoklav wird mit 100 Teilen Kaliumformiat befüllt und bei 230°C die Schmelze unter Rühren im Vakuum von Restfeuchte befreit. Danach wird auf 280°C aufgeheizt und 50 bar Kohlenmonoxid aufgedrückt. Mit einer Dosierpumpe wird innerhalb von 5 Stunden eine Mischung von 20 Teilen Kaliumnaphtholat mit 25 Teilen Kaliumcarbonat als Suspension in Kerosin eindosiert. Kerosin wird dabei teilweise aus dem Reaktionsgefäß bei vermindertem Druck abdestilliert, kondensiert und ausgeschleust. Nach dem Abkühlen wird die Reaktionsmischung in Wasser aufgenommen und Kerosin als Oberphase abdekantiert. Nach Ansäuerung mit Schwefelsäure auf einen pH-Wert von 7 wird das unumgesetzte β-Naphtholat gefällt und abgetrennt. Bei weiterer Ansäuerung auf pH 4 fällt zunächst die 2-Hydroxy-naphthalin-6-carbonsäure und schließlich bei pH 1 die übrigen Säuren aus. Nach Aufarbeitung des Reaktionsansatzes erhält man die 2-Hydroxy-naphthalin-6-carbonsäure in einer Ausbeute von 72 % bezogen auf eingesetztes Kaliumnaphtholat. Daneben fallen 8 % unumgesetztes β-Naphthol, 4 % 2-Hydroxy-naphthalin-3-carbonsäure und 12 % 2-Hydroxynaphthalin-3,6-dicarbonsäure an.

### Beispiel 2

Ein Edelstahl-Druckautoklav wird mit 50 Teilen Kerosin und 10 Teilen Kaliumformiat befüllt und auf 280°C aufgeheizt. Danach werden 50 bar Kohlenmonoxid aufgedrückt. Über eine Dosierpumpe werden im Verlaufe von 5 Stunden eine Mischung bestehend aus 50 Teilen Kaliumnaphtholat und 38 Teilen Kaliumcarbonat als Suspension in Kerosin in den Reaktor eindosiert. Kerosin wird dabei teilweise aus dem Reaktionsgefäß bei erniedrigtem Druck abdestilliert, kondensiert und ausgeschleust. Nach dem üblichen Aufarbeiten wird 2-Hydroxy-naphthalin-6-carbonsäure in einer Ausbeute von 67 %, bezogen auf eingesetztes K-Naphtholat, erhalten. Daneben fallen 15 % unumgesetztes β-Naphthol, 4 % 2-Hydroxy-naphthalin-3-carbonsäure sowie 9 % 2-Hydroxy-naphthalin-3,6-dicarbonsäure an.

## Patentansprüche

1. Verfahren zur Herstellung von aromatischen Hydroxycarbonsäuren durch Umsetzung von Alkaliphenolaten oder Alkalinaphtholaten mit Alkalicarbonaten und Kohlenmonoxid und anschließendes Ansäuern, dadurch gekennzeichnet, daß die festen Edukte Alkalicarbonat und/oder Alkali-phenolat oder - naphtholat in Form einer oder mehrerer Dispersionen in einer inerten organischen Flüssigkeit in die Reaktionsmischung zudosiert werden.

2. Verfahren nach Anspruch 1, dadurch gekennzeichnet, daß die Dispersion über den gesamten Reaktionsverlauf zudosiert wird.

3. Verfahren nach Anspruch 1 oder 2, dadurch gekennzeichnet, daß die Dispersion kontinuierlich zugegeben wird.

4. Verfahren nach Anspruch 1 oder 2, dadurch gekennzeichnet, daß die Dispersion getaktet zugegeben wird.

5. Verfahren nach einem oder mehreren der vorhergehenden Ansprüche, dadurch gekennzeichnet, daß als Phenolat oder Naphtholat eine Verbindung der Formel (I) eingesetzt wird, 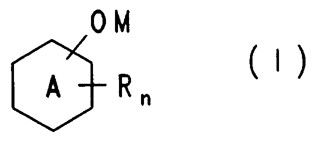 worin die Symbole und Indizes folgende Bedeutungen haben: 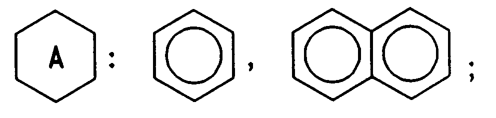
M : Li, Na, K
R : unabhängig voneinander, OM, COOM, F, Cl, Br, I, NH₂, CF₃, eine Alkylgruppe mit 1 bis 6 C-Atomen, eine Alkoxygruppe mit 1 bis 6 C-Atomen;
n : 0, 1, 2, 3, 4.

6. Verfahren nach Anspruch 5, dadurch gekennzeichnet, daß als Alkalinaphtholat Kalium-β-naphtholat eingesetzt wird.

7. Verfahren nach einem oder mehreren der vorhergehenden Ansprüche, dadurch gekennzeichnet, daß die inerte organische Flüssigkeit ein aliphatischer oder aromatischer Kohlenwasserstoff oder Ether bzw. eine Mischung aus mindestens zwei solcher Verbindungen ist.

8. Verfahren nach einem oder mehreren der vorhergehenden Ansprüche, dadurch gekennzeichnet, daß die Dispersion 5 bis 50 Gew.-% ,bezogen auf die gesamte Dispersion, an Alkalicarbonat enthält.

9. Verfahren nach einem oder mehreren der vorhergehenden Ansprüche, dadurch gekennzeichnet, daß die Dispersion 5 bis 70 Gew.-%, bezogen auf die gesamte Dispersion, an Alkaliphenolat oder Alkalinaphtholat enthält.

10. Verfahren nach einem oder mehreren der vorhergehenden Ansprüche, dadurch gekennzeichnet, daß die eingesetzten Feststoffe eine mittlere Korngröße von weniger als 50 µm haben.

11. Verfahren nach einem oder mehreren der vorhergehenden Ansprüche, dadurch gekennzeichnet, daß Kaliumcarbonat und Phenolat oder Naphtholat gemeinsam in Form einer Dispersion in das Reaktionsgemisch eingebracht werden.

12. Verfahren nach einem oder mehreren der Ansprüche 1 bis 10, dadurch gekennzeichnet, daß Alkalicarbonat und Alkaliphenolat oder Naphtholat jeweils getrennt als Dispersion in das Reaktionsgefäß eingebracht werden.

13. Mischung, enthaltend
a) 5 bis 50 Gew.-% Alkalicarbonat und/oder
b) 5 bis 70 Gew.-% eines Alkaliphenolats oder Alkalinaphtholats und
c) 5 bis 95 Gew.-% eines aliphatischen oder aromatischen Kohlenwasserstoffes oder eines aliphatischen oder aromatischen Ethers oder einer Mischung aus mindestens zwei dieser Verbindungen.

14. Mischung nach Anspruch 13, dadurch gekennzeichnet, daß das Alkalicarbonat und/oder Phenolat oder Naphtholat eine mittlere Korngröße von weniger als 50 µm aufweist.
